# NEW EUROPEAN PATENT SPECIFICATION

(11) **EP 3 746 020 B2**
(45) Date of publication and mention of the opposition decision: **30.09.2026**
(45) Mention of the grant of the patent: 23.08.2023
(21) Application number: 19700217.3
(22) Date of filing: 08.01.2019
(51) Int. Cl.: A61F 13/02

(54) **WOUND DRESSING**
WUNDAUFLAGE
PANSEMENT

(30) Priority: 31.01.2018 DE 102018102126
(43) Date of publication of application: 09.12.2020
(73) Proprietor: Lohmann & Rauscher GmbH & Co. KG, 56567 Neuwied (DE)
(72) Inventor: DODEL, Kirsten, 56566 Neuwied (DE); POREMBA, Marc, 56428 Dernbach (DE); WALGENBACH, Andreas, 53567 Buchholz (DE); SANDHOF, Marc, 56579 Rengsdorf (DE)
(74) Representative: Herrmann, Daniel
(86) International application number: PCT/EP2019/050277
(87) International publication number: WO 2019/149476

(56) References cited:
- EP-A1- 2 338 529
- EP-B1- 0 769 283
- WO-A1-2010/048480
- WO-A1-2012/150235
- WO-A1-2015/059501
- WO-A1-2015/124936
- WO-A1-2017/081469
- WO-A1-95/14451
- WO-A1-98/25559
- BE-A3- 1 019 122
- US-A- 4 915 102
- US-A- 5 628 724
- US-A1- 2004 126 413
- US-A1- 2010 179 463
- US-B2- 7 982 087
- US-B2- 9 820 888

## Description

The invention relates to a wound dressing as specified in the pre-characterizing portion of appended claim 1.

Such dressings are used to treat conventional wounds and burns. In this case exudate exiting the wound is taken up by the wound contact layer. In modern wound dressings, the wound contact layer may comprise a hydrogel or a silica gel, whereby the gel swells due to ingestion of liquid, but does not dissolve. The use of appropriate wound contact layers has the advantage that a gel may both accommodate or absorb, as well as release liquids, so as to adjust a desired moisture milieu in the area of the wound. This can promote the healing process.

Hydrogels which can be used as a wound contact layer are disclosed in WO 2015/059501 A1. The disclosure of this document with respect to the composition of hydrogels and additives for hydrogels is hereby incorporated by express reference into this specification.

In the EP 2 410 962 B1 it is proposed to improve the structural integrity of a hydrogel layer by means of a reinforcing layer. However, the wearing comfort of corresponding wound dressings is impaired by the reinforcing layer. The wearing comfort of wound dressings with wound contact layers having a hydrogel is in any case impaired by the swelling of the wound contact layer when liquids are taken up.

The wound dressing is usually fixed with an adhesive to the skin surrounding the wound. The adhesive may in this case be provided in a region of the wound surrounding the wound contact layer being on the side of the support layer facing the wound. In many cases, the carrier layer is fully coated with the adhesive. The adhesive also serves to fix the wound contact layer on the carrier layer.

The wearing comfort of the wound dressing just described with a wound contact layer comprising a hydrogel or a silica gel, according to WO 2015/059501 A1, is improved by the wound contact layer being formed by discrete hydrogel islands, which are mounted at a distance from each other on the carrier layer. Such wound dressings of the type mentioned above can then be adapted to the wound or the skin surrounding the wound by adopting a desired shape. This may result in a satisfactory wearing comfort.

When using appropriate wound dressings, however, it may happen that individual hydrogel islands detach from the carrier layer and contaminate the wound. According to WO 2015/059501 A1, in view of this deficiency, an additional reinforcing layer can be incorporated into the individual hydrogel islands. Additionally or alternatively, a continuous absorption layer without intermediate spaces between individual wound contact surfaces can be applied to the end faces of the hydrogel islands facing away from the carrier layer. This, in turn, reduces the wearing comfort of these wound dressings. In addition, the production process for such wound dressings is complicated.

A wound dressing as specified in the pre-characterizing portion of claim 1 is disclosed in WO 95/14451.

In view of the above problems in the prior art, the invention has for its object to provide a wound dressing which on the one hand offers a satisfactory wearing comfort and on the other hand is easy to produce.

According to the invention, this object is achieved by a further development of the known wound dressings as specified in the characterizing portion of appended claim 1.

In this way, a wound contact layer is obtained, which has an overall coherent or contiguous topology. This avoids that individual wound contact layer islands can be easily detached from the carrier layer. Due to the distance between the wound contact surfaces, the wound contact layer, even in the swollen state in which it has already absorbed exudate, has sufficient flexibility to adapt it to the shape of the wound or of the skin surrounding the wound. The contact with the wound takes place at least in the area of the wound contact surfaces. In this area, the wound exudate can be absorbed directly by the wound contact layer. However, the wound exudate can also penetrate into the spaces between the wound contact surfaces and penetrate from there via lateral boundary surfaces into the wound contact layer and be absorbed there.

In a preferred embodiment of the invention, the wound contact layer in at least one connection region has a smaller thickness in a direction perpendicular to the carrier layer than in the region of the contact surfaces. In this embodiment of the invention, the carrier layer in the area of the gaps is covered by the wound contact layer. However, through the lower thickness of the wound contact layer in this connection area a desired bending portion of the wound contact layer is provided which favors pliability of the wound contact layer and thus of the entire wound dressing. In this embodiment exudate penetrating into the interspaces between the wound contact surfaces can already be accommodated in the region of the interstices of the wound contact layer. The wound contact surfaces can be provided by a knob- or nap-shaped structure of the wound contact layer.

In addition or as an alternative to the embodiment of the invention just described, the wound contact layer in at least one connection region may have approximately the same thickness as in the region of the wound contact surfaces and also in contact with the wound in this connection region. In this case, the wound contact surfaces, which are separated from one another by the intermediate spaces, in which the wound contact layer is not in contact with the wound, are connected to one another by the connecting areas which may also come into contact with the wound, wherein the gaps, in which the side of the support layer facing the wound is exposed, also provide desired bending points of the entire wound dressing, which favor the flexibility of the wound dressing as a whole.

The interstices between individual wound contact surfaces with wound dressings according to the invention may be realized by wound contact layers which are at least partially concavely bounded. In the context of this invention, a topology of the wound contact layer is described by "concavely bounded" in cases where individual points on the edges of the wound contact layer are connected to each other at least in sections by straight lines running outside the wound contact layer. A concave boundary can also be provided by, for example, the wound contact layer as a whole being bounded in a bone or eight shape. In a preferred embodiment of the invention, at least two wound contact surfaces are at least partially approximately parallel to each other and preferably formed by approximately rectilinear wound contact strips. Between the wound contact strips a gap is formed, in which the wound-facing boundary surface of the carrier layer is not covered by the wound contact layer. This means that a concave boundary according to the invention can be provided both by rounded edges as well as by straight edge portions, as well as by combinations of rounded edge portions with straight edge portions.

The distance between the wound contact strips may be 0.5 mm or more. However, to provide sufficient absorption performance, the distance between the wound contact strips is desirably 30 mm or less, more preferably 20 mm or less, more preferably 10 mm or less. The spaced wound contact strips may enclose an acute angle of 20° or less.

In order to ensure the desired structural integrity of the wound contact layer while at the same time providing adequate flexibility of the wound contact layer, it has proved favorable if the wound contact strips emanate from a common wound contact bar, which preferably extends perpendicular to the wound contact strips, wherein the wound contact layer may also come into contact with the wound in the area of the wound contact bar. Additionally or alternatively, the wound contact bar can be made with a smaller thickness and form a gap between wound contact surfaces.

At least two wound contact strips can extend from the wound contact bar in opposite directions. In the context of the invention, it is also contemplated that wound contact strips and wound contact bar form a herringbone pattern with each other, in which the wound contact strips include an angle of less than 90°, but preferably more than 45° with the wound contact bar.

In addition or as an alternative to the embodiment of the invention just described, at least one wound contact strip may have at its one end a preferably curved connection region with a first adjacent wound contact strip approximately parallel or at an acute angle thereto and at its other end another, preferably also approximately curved executed connection region with a second adjacent wound contact strip disposed on the side facing away from the first adjacent wound contact strip . As mentioned above, the individual wound contact strips can be made approximately parallel to one another or at an acute angle.

As already explained above in connection with wound dressings according to the prior art, the wound contact layer comprises or consists of a hydrogel and/or silica gel. Hydrogels which can be used in the context of the invention are described in WO 2015/059501 A1.

As with conventional wound dressings, an adhesive layer is arranged between the carrier layer and the wound contact layer. In this case, the adhesive layer also at least partially covers a region of the carrier layer that surrounds the wound contact layer so as to enable the wound dressing to be attached to the skin surrounding the wound.

To avoid premature bonding of the adhesive layer, this layer and/or the wound contact layer can be covered by at least one cover strip detachably disposed thereon, such as a dehesively finished, for example Si-coated, waterproof film and/or silicone paper . In order to further improve the wearing comfort of wound dressings according to the invention, it can be provided that the carrier layer has or consists of a preferably transparent PU film. Such a polyurethane film can be made very thin, permeable to water vapor, and pliable, so as to allow optimum adaptation of the wound dressing to the shape of the wound and the skin surrounding the wound.

The application of wound dressings according to the invention can be facilitated if a support layer, in particular a support film, which is detachably attached to the side of the carrier layer facing away from the wound contact layer, is provided. In this case, the support film may preferably be connected via at least one film hinge with at least one cover strip. For application of wound dressings according to the invention, in the last-described embodiment of the invention the cover strip can first be removed from the wound dressing's boundary surface, then the wound dressing can be fixed on the wound or the skin surrounding the wound with the aid of the adhesive layer. Subsequently, the cover strip can be used as an aid to the detachment of the support layer or the support film so as to release the support layer from the carrier layer already fixed to the wound or the skin surrounding the wound.

In the following, the invention will be explained with reference to the drawing, to which reference is expressly made with regard to all details essential to the invention and details which are not further detailed in the description. In the drawing shows:
- Fig. 1: a first embodiment of a wound dressing according to the invention,
- Fig. 2: a second embodiment of a wound dressing according to the invention,
- Fig. 3: a third embodiment of a wound dressing according to the invention and
- Fig. 4: A fourth embodiment of a wound dressing according to the invention.

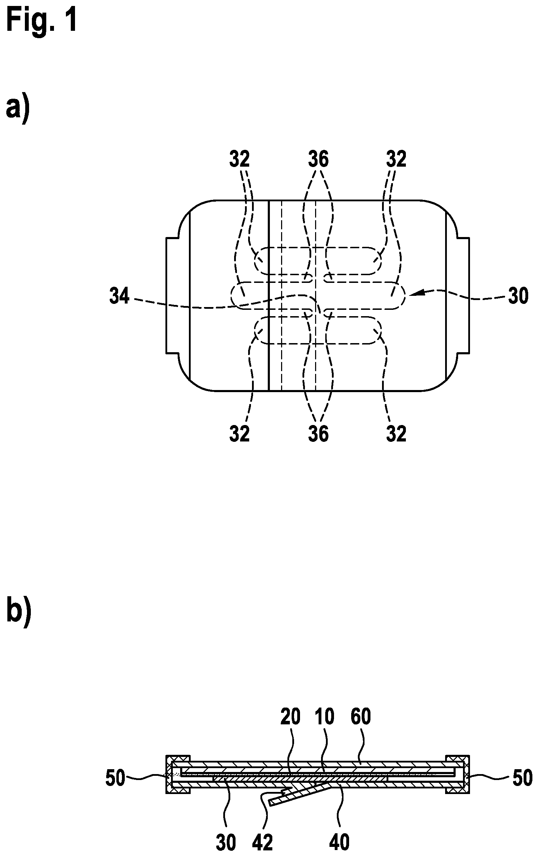

In Fig. 1a) a top view of a wound dressing according to the invention is shown. Fig. 1b) shows a sectional view of the wound dressing shown in Fig. 1a). According to Fig. 1b), the wound dressing comprises a carrier layer 10, on which a wound contact layer 30 is fixed with the aid of an adhesive layer 20. The adhesive layer 20 circumscribes the wound contact layer 30 and thus makes it possible to fix the wound dressing in this peripheral adhesive area on the skin surrounding the wound. According to Fig. 1b), the wound contact layer 30 is covered by cover strips 40 and 42 on its side facing away from the carrier layer 10. The cover strips 40 and 42 are connected by hinges 50, which run around edges of the carrier layer 10, to a support film 60.

For application of the wound dressing illustrated in Fig. 1b), the cover strips 40 and 42 are released from the wound contact layer 30 or the surrounding adhesive region 12. The wound dressing is then fixed with the aid of the adhesive layer 20 on the skin surrounding the wound. Following this, the cover strips 40 and 42 can be used as grasping aid with which the support film 60 connected thereto via the hinge joints 50 can be removed from the side of the carrier layer 10 facing away from the wound.

According to Fig. 1a), the wound contact layer 30 has a total of six wound contact strips 32, which are spaced apart from one another by gaps 36. In the area of the intermediate spaces 36, the carrier layer 10 is not covered by the wound contact layer 30. The wound contact strips 32 extend approximately parallel to each other and extend from a wound contact bar 34 in opposite directions approximately parallel to the edges of the carrier layer 10. The wound contact layer 30 according to the embodiment of the invention shown in Fig. 1 has in all areas a thickness of about 0.5 to 1.5 mm, in particular about 1 mm. In use it preferably lies completely against the wound on its side facing the wound.

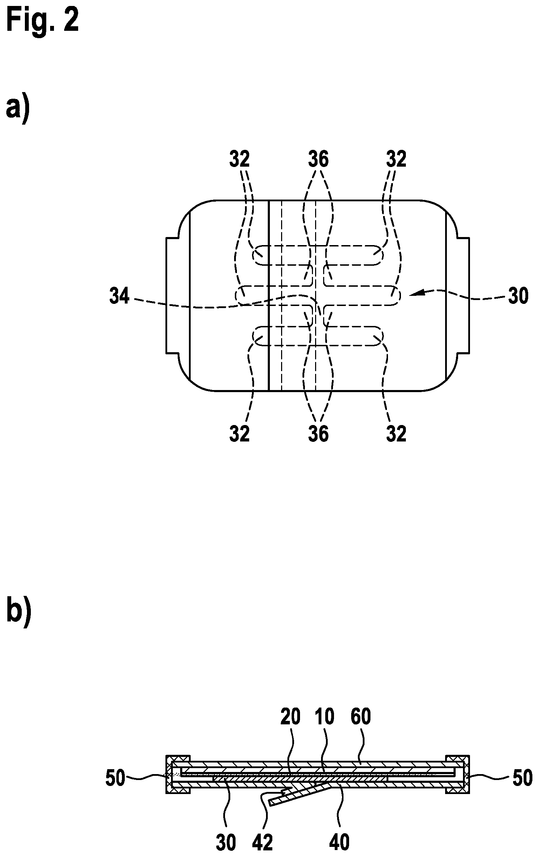

The embodiment of the invention illustrated in Fig. 2 differs essentially from the embodiment shown in Fig. 1 in that the wound contact strips are 32 narrower and the interstices, in the region of which the substrate layer is not covered by the wound contact layer, are wider. As a result, a better flexibility of the wound dressing is achieved.

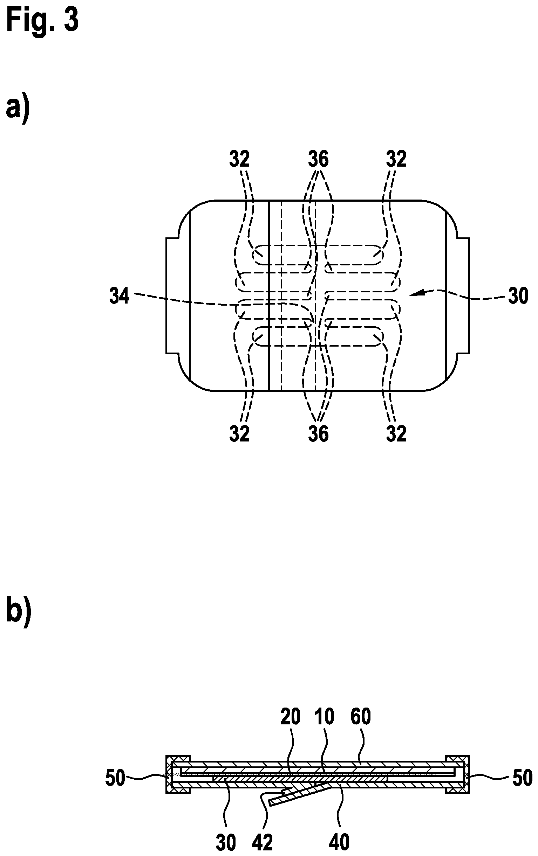

The embodiment of the invention illustrated in Fig. 3 essentially differs from the embodiment explained with reference to Fig. 1 in that a total of eight narrow contact strips extend in mutually opposite directions approximately parallel to one another starting from a wound contact bar 34. In this embodiment too, the flexibility of the wound dressing is increased by increasing the number of intermediate spaces in which the carrier layer is not covered by the wound contact layer. In addition, by increasing the number of strips in the in Fig. 3 embodiment, the absorption capacity can be increased.

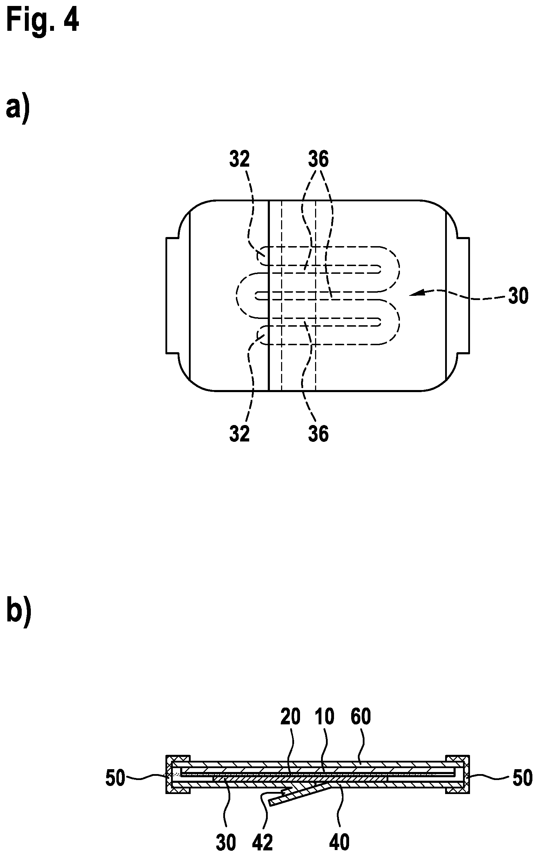

In the embodiment of the invention shown in Fig. 4, the wound contact layer is made approximately meander-shaped. A total of four wound contact strips 32 are connected to one another via a total of three connection regions. The middle wound contact strips are connected at their one end via a curved connection region to a first adjacent wound contact strip extending approximately parallel thereto and at another end via a further connection region to a second adjacent wound contact strip arranged at a side of the at least one wound contact strip opposite to the first adjacent wound contact strip.

The invention is not limited to the embodiments explained with reference to the drawing. It is also thought of wound contact layers revolving spirally. Two, three or more wound contact bars can be provided, via which the wound contact strips can be connected to one another. It is essential that the wound contact layer as a whole constitutes a coherent structure which is fixed to the carrier layer and forms intermediate spaces in which no contact of the wound contact layer with the wound takes place.

## Claims

1. Wound dressing with a carrier layer and an absorbent wound contact layer (30) fixed to the carrier layer (10), which has wound contact surfaces spaced from one another by interstices (36), in which the absorbent wound contact layer (30) has no contact with the wound wherein the wound contact surfaces are connected to each other by connection regions (34) of the absorbent wound contact layer (30), wherein the absorbent wound contact layer (30) comprises or is a hydrogel and/or silica gel, **characterized in that** in the area of the interstices (36) the carrier layer (10) is not covered by the absorbent wound contact layer (30); an adhesive layer (20) is arranged between the carrier layer (10) and the absorbent wound contact layer (30), wherein the adhesive layer (20) at least partially covers a region of the carrier layer (10) that surrounds the wound contact layer (30) so as to enable the wound dressing to be attached to skin surrounding the wound.

2. Wound dressing according to claim 1, **characterized in that** the absorbent wound contact layer (30) in at least one connecting region (34) has a smaller thickness in a direction perpendicular to the carrier layer plane than in the region of the contact surfaces.

3. Wound dressing according to one of the preceding claims, **characterized in that** the absorbent wound contact layer (30) has approximately the same thickness in at least one connecting region (34) as in the region of the wound contact surface and also comes into contact with the wound in this region.

4. Wound dressing according to one of the preceding claims, **characterized in that** the absorbent wound contact layer (30) is at least partially bounded concavely.

5. Wound dressing according to one of the preceding claims, **characterized in that** at least two wound contact surfaces are at least partially approximately parallel to each other.

6. Wound dressing according to claim 5, **characterized in that** the wound contact surfaces are formed by approximately rectilinear wound contact strips and the distance between the wound contact strips is 0.5 mm or more, and / or 10 mm or less.

7. Wound dressing according to claim 5 or 6, **characterized in that** the wound contact strips (32) emanate from a common wound contact bar (34.

8. Wound dressing according to claim 7, **characterized in that** at least two wound contact strips (32) emanating from the wound contact bar (34) extend in opposite directions.

9. Wound dressing according to one of claims 5 to 8, **characterized in that** the wound contact surfaces are formed by approximately rectilinear wound contact strips and at least one wound contact strip (32) is connected at its one end via a connection region to a first adjacent wound contact strip (32) and at its other end via a further connection region to a second adjacent wound contact strip (32) arranged at a side of the at least one wound contact strip (32) opposite to the first adjacent wound contact strip (32).

10. Wound dressing according to one of the preceding claims, **characterized in that** the adhesive layer (20) and/or the absorbent wound contact layer (30) is covered by at least one cover strip (40, 42) detachably mounted thereon.

11. Wound dressing according to one of the preceding claims, **characterized in that** the carrier layer (10) comprises or consists of a PU film.

12. Wound dressing according to one of the preceding claims, **characterized by** a support layer (60), in particular a support film, detachably mounted on the side of the carrier layer (10) facing away from the wound contact layer (30).

13. Wound dressing according to claim 12, **characterized in that** the support film (60) is preferably connected via at least one film hinge (50) with at least one cover strip (40, 42).

## Patentansprüche

1. Wundauflage mit einer Trägerschicht und einer an der Trägerschicht (10) befestigten saugfähigen Wundkontaktschicht (30), die durch Zwischenräume (36) voneinander beabstandete Wundkontaktflächen aufweist, wobei die saugfähige Wundkontaktschicht (30) keinen Kontakt zur Wunde aufweist, wobei die Wundkontaktflächen durch Verbindungsbereiche (34) der saugfähigen Wundkontaktschicht (30) miteinander verbunden sind, wobei die saugfähige Wundkontaktschicht (30) ein Hydrogel und/oder Silicagel umfasst oder ist, **dadurch gekennzeichnet, dass** die Trägerschicht (10) im Bereich der Zwischenräume (36) nicht von der saugfähigen Wundkontaktschicht (30) bedeckt ist;
zwischen der Trägerschicht (10) und der saugfähigen Wundkontaktschicht (30) ist eine Klebstoffschicht (20) angeordnet, wobei die Klebstoffschicht (20) einen die Wundkontaktschicht (30) umgebenden Bereich der Trägerschicht (10) zumindest teilweise bedeckt, um eine Befestigung der Wundauflage an die Wunde umgebender Haut zu ermöglichen.

2. Wundauflage nach Anspruch 1, **dadurch gekennzeichnet, dass** die saugfähige Wundkontaktschicht (30) in zumindest einem Verbindungsbereich (34) in einer Richtung senkrecht zur Trägerschichtebene eine geringere Dicke aufweist als im Bereich der Kontaktflächen.

3. Wundauflage nach einem der vorhergehenden Ansprüche, **dadurch gekennzeichnet, dass** die saugfähige Wundkontaktschicht (30) in zumindest einem Verbindungsbereich (34) in etwa die gleiche Dicke aufweist wie im Bereich der Wundkontaktfläche und auch in diesem Bereich mit der Wunde in Kontakt kommt.

4. Wundauflage nach einem der vorhergehenden Ansprüche, **dadurch gekennzeichnet, dass** die saugfähige Wundkontaktschicht (30) zumindest teilweise konkav begrenzt ist.

5. Wundauflage nach einem der vorhergehenden Ansprüche, **dadurch gekennzeichnet, dass** zumindest zwei Wundkontaktflächen zumindest teilweise in etwa parallel zueinander verlaufen.

6. Wundauflage nach Anspruch 5, **dadurch gekennzeichnet, dass** die Wundkontaktflächen durch in etwa geradlinige Wundkontaktstreifen gebildet sind und der Abstand zwischen den Wundkontaktstreifen 0,5 mm oder mehr und/oder 10 mm oder weniger beträgt.

7. Wundauflage nach Anspruch 5 oder 6, **dadurch gekennzeichnet, dass** die Wundkontaktstreifen (32) von einem gemeinsamen Wundkontaktsteg (34 ausgehen.

8. Wundauflage nach Anspruch 7, **dadurch gekennzeichnet, dass** zumindest zwei von dem Wundkontaktsteg (34) ausgehende Wundkontaktstreifen (32) in entgegengesetzte Richtungen verlaufen.

9. Wundauflage nach einem der Ansprüche 5 bis 8, **dadurch gekennzeichnet, dass** die Wundkontaktflächen durch in etwa geradlinige Wundkontaktstreifen gebildet sind und zumindest ein Wundkontaktstreifen (32) an seinem einen Ende über einen Verbindungsbereich mit einem ersten benachbarten Wundkontaktstreifen (32) und an seinem anderen Ende über einen weiteren Verbindungsbereich mit einem zweiten benachbarten Wundkontaktstreifen (32) verbunden ist, der an einer dem ersten benachbarten Wundkontaktstreifen (32) gegenüberliegenden Seite des zumindest einen Wundkontaktstreifens (32) angeordnet ist.

10. Wundauflage nach einem der vorhergehenden Ansprüche, **dadurch gekennzeichnet, dass** die Klebstoffschicht (20) und/oder die saugfähige Wundkontaktschicht (30) von zumindest einem daran lösbar angebrachten Abdeckstreifen (40, 42) bedeckt ist.

11. Wundauflage nach einem der vorhergehenden Ansprüche, **dadurch gekennzeichnet, dass** die Trägerschicht (10) eine PU-Folie umfasst oder daraus besteht.

12. Wundauflage nach einem der vorhergehenden Ansprüche, **gekennzeichnet durch** eine auf der der Wundkontaktschicht (30) abgewandten Seite der Trägerschicht (10) lösbar angebrachte Trägerschicht (60), insbesondere eine Trägerfolie.

13. Wundauflage nach Anspruch 12, **dadurch gekennzeichnet, dass** die Trägerfolie (60) vorzugsweise über zumindest ein Filmscharnier (50) mit zumindest einem Abdeckstreifen (40, 42) verbunden ist.

## Revendications

1. Pansement comprenant une couche de support et une couche de contact avec la plaie absorbante (30) fixée sur la couche de support (10), qui présente des surfaces de contact avec la plaie espacées les unes des autres par des interstices (36), dans lequel la couche de contact avec la plaie absorbante (30) n'a pas de contact avec la plaie, dans lequel les surfaces de contact avec la plaie sont reliées les unes aux autres par des zones de liaison (34) de la couche de contact avec la plaie absorbante (30), dans lequel la couche de contact avec la plaie absorbante (30) comprend ou est un hydrogel et/ou un gel de silice, **caractérisé en ce que** dans la zone des interstices (36), la couche de support (10) n'est pas recouverte par la couche de contact avec la plaie absorbante (30);
une couche adhésive (20) est agencée entre la couche de support (10) et la couche de contact avec la plaie absorbante (30), dans lequel la couche adhésive (20) recouvre au moins partiellement une zone de la couche de support (10) qui entoure la couche de contact avec la plaie (30) afin de permettre au pansement d'être fixé sur la peau entourant la plaie.

2. Pansement selon la revendication 1, **caractérisé en ce que** la couche de contact avec la plaie absorbante (30) présente dans au moins une zone de liaison (34) une plus petite épaisseur dans une direction perpendiculaire au plan de la couche de support que dans la zone des surfaces de contact.

3. Pansement selon l'une quelconque des revendications précédentes, **caractérisé en ce que** la couche de contact avec la plaie absorbante (30) présente approximativement la même épaisseur dans au moins une zone de liaison (34) que dans la zone de la surface de contact avec la plaie et vient également en contact avec la plaie dans cette zone.

4. Pansement selon l'une quelconque des revendications précédentes, **caractérisé en ce que** la couche de contact avec la plaie absorbante (30) est au moins partiellement délimitée de manière concave.

5. Pansement selon l'une quelconque des revendications précédentes, **caractérisé en ce qu'**au moins deux surfaces de contact avec la plaie sont au moins partiellement approximativement parallèles l'une à l'autre.

6. Pansement selon la revendication 5, **caractérisé en ce que** les surfaces de contact avec la plaie sont formées par des bandes de contact avec la plaie approximativement rectilignes et la distance entre les bandes de contact avec la plaie est de 0,5 mm ou plus et/ou de 10 mm ou moins.

7. Pansement selon la revendication 5 ou 6, **caractérisé en ce que** les bandes de contact avec la plaie (32) partent d'une barre commune de contact avec la plaie (34).

8. Pansement selon la revendication 7, **caractérisé en ce qu'**au moins deux bandes de contact avec la plaie (32) partant de la barre de contact avec la plaie (34) s'étendent dans des directions opposées.

9. Pansement selon l'une quelconque des revendications 5 à 8, **caractérisé en ce que** les surfaces de contact avec la plaie sont formées par des bandes de contact avec la plaie approximativement rectilignes et au moins une bande de contact avec la plaie (32) est reliée à une de ses extrémités par une zone de liaison à une première bande de contact avec la plaie adjacente (32) et à son autre extrémité par une autre zone de liaison à une deuxième bande de contact avec la plaie adjacente (32) agencée sur un côté de l'au moins une bande de contact avec la plaie (32) opposé à la première bande de contact avec la plaie adjacente (32).

10. Pansement selon l'une quelconque des revendications précédentes, **caractérisé en ce que** la couche adhésive (20) et/ou la couche de contact avec la plaie absorbante (30) est recouverte par au moins une bande de recouvrement (40, 42) montée de manière détachable sur celle-ci.

11. Pansement selon l'une quelconque des revendications précédentes, **caractérisé en ce que** la couche de support (10) comprend ou est constituée d'un film de PU.

12. Pansement selon l'une quelconque des revendications précédentes, **caractérisé par** une couche de support (60), en particulier un film de support, montée de manière détachable sur le côté de la couche de support (10) opposé à la couche de contact avec la plaie (30).

13. Pansement selon la revendication 12, **caractérisé en ce que** le film de support (60) est de préférence relié par au moins une charnière à film (50) à au moins une bande de recouvrement (40, 42).
